# EUROPEAN PATENT SPECIFICATION

(11) **EP 1 579 875 B1**
(45) Date of publication and mention of the grant of the patent: **13.08.2008**
(21) Application number: 05006656.2
(22) Date of filing: 24.03.2005
(51) Int. Cl.: A61L 27/06, A61L 27/36, A61C 8/00, A61F 2/00, A61F 2/30

(54) **Implant made with titanium or titanium alloy and surface treating method thereof**
Implantat aus Titan oder einer Titanlegierung und Verfahren zu seiner Oberflächenbehandlung
Implant fabriqué de titane ou alliage de titane et son procédé de traitement de surface

(30) Priority: 25.03.2004 JP 2004088551
(43) Date of publication of application: 28.09.2005
(73) Proprietor: GC Corporation, Itabashi-ku Tokyo 174-8585 (JP)
(72) Inventor: Kuboki, Takuo, Okayama-shi, Okayama (JP); Maekawa, Kenji, Okayama-shi, Okayama (JP); Yoshida, Yasuhiro, Okayama-shi, Okayama (JP); Mine, Atsushi, Okayama-shi, Okayama (JP); Fujisawa, Takuo, Okayama-shi, Okayama (JP); Suzuki, Kazuomi, Okayama-shi, Okayama (JP); van Meerbeek, Bart, Heverlee 3001 (BE); Kaneko, Tadashi, Itabashi-ku, Tokyo (JP)
(74) Representative: Müller-Boré & Partner Patentanwälte

(56) References cited:
- CN-A- 1 451 367
- GB-A- 1 401 233
- US-A1- 2003 135 282
- PULEO D A ET AL: "A technique to immobilize bioactive proteins, including bone morphogenetic protein-4 (BMP-4), on titanium alloy" May 2002 (2002-05), BIOMATERIALS, ELSEVIER SCIENCE PUBLISHERS BV., BARKING, GB, PAGE(S) 2079-2087 , XP004345254 ISSN: 0142-9612 * abstract *
- LIU X ET AL: "Surface modification of titanium, titanium alloys, and related materials for biomedical applications" MATERIALS SCIENCE AND ENGINEERING R: REPORTS, ELSEVIER SEQUOIA S.A., LAUSANNE, CH, vol. 47, no. 3-4, 24 December 2004 (2004-12-24), pages 49-121, XP004722113 ISSN: 0927-796X

## Description

The present invention relates to a titanium or titanium alloy implant, and a surface treating method thereof. More particularly, the present invention relates to the titanium or titanium alloy implant obtainable by a process, in which the surface is treated in order to make a bone bonding promptly, and the surface treating method thereof.

An implant treatment is widely used as one choice of a dental prosthesis treatment. As a main factor of a result in the dental prosthesis treatment, there is the quality of acquiring "the bone bonding". A fixture of the implant is incorporated with jaw bone by the bone bonding and then, a dental prosthesis is fixed at upper part of the fixture to have a function for the dental prosthesis. However, in order to obtain the bone bonding in general, it is necessary to put the fixture under a gum with the quiet state for a long period of time of 3 to 4 months at a lower jaw and 6 months at an upper jaw. Now, under increasing tendency that the implant treatment must be considered to focus on a patient's position, it is an objective to shorten the period of the bone bonding such as 3 to 6 months.

Thus, it is remarkably important to make the bone bonding in an early stage, and a modification of a surface of an implant material becomes to be the technical main point for making the early bone bonding. The titanium and the titanium alloy are applied most widely as the implant material in a clinical case. It is considered now that biocompatibilities of the titanium and the titanium alloy originate in a dense and strong oxide film (TiO₂), which is called as a passivation film and formed on the surface thereof. This film has not only high corrosion resistance with respect to a chemical invasion but also has fully dielectric constant for obtaining the strong bonding with biotissues. As a treatment for modifying the surface characteristic, that is, "a surface modification" to obtain the bone bonding in the early stage while the characteristics of the titanium and the titanium alloy being kept, the following treatments are used. A surface treatment for depositing apatite (for example, refer to Japanese Patent Laid Open No. 05-285212, 05-285213, 08-299429, 10-108905), a surface treatment for carrying out a plasma coating (for example, refer to Filiaggi, M.J., Coombs, N.A. and Pilliar, R.M. : Characterization of the interface in the plasma-sprayed HA coating/Ti-6A1-4V implant system. J. Biomed. Mater Res. 25, 1211-1229, 1991), a surface treatment for carrying out a calcium phosphate coating (for example, refer to Hulshoff, J.E.G., Dijk, K., de Ruijter, J.E., Rietveld, F.J.R., Ginsel, L.A. and Jansen, J.A. Interfacial phenomena: An in vitro study of the effect of calcium phosphate (Ca-P) ceramic on bone formation. J. Biomed. MaterRes. 40, 464-474, 1998), a surface treatment in which a sandblast and an acid treatment are used together (for example, refer to Carlsson, L., Rostlund, T., Albrektsson, B. and Albrektsson, T. : Removal torques for polished and rough titanium implants. Int. J. Oral Maxillofac. Implants 3, 21-24, 1998), and a surface treatment with a hydrothermal alkali treatment (for example, refer to Yan, W.Q., Nakamura, T., Kobayashi, M., Kim, H-M., Miyaji, F. and Kokubo, T. : Bonding of chemically treated titanium implants to bone. J. Biomed. Mater Res. 37, 267-275, 1997). However, these conventional surface modifications have problems in which an interface between the coating material and titanium or the titanium alloy or the coating material itself is easily broken, and the effects of these surface modifications are insufficient. Thus, it cannot be said that the sufficient bone bonding can be realized fully in the early stage.

Then, in recent years, the surface modification by biofunctional protein is noticed, where this biofunctional protein induces the tissue reproduction of a cell bonding protein, a structural protein or the like. The methods carried out are for example, a method in which an osteoblast is coated on the surface (for example, refer to Japanese Patent Laid Open No. 2001-333974), a method in which calcium or phosphorus and an enzyme is coated on the surface (for example, refer to Japanese Patent Laid Open No. 05-023361), a method in which a peptide is bonded on the surface of titanium or the titanium alloy after carrying out a silane treatment (for example, refer to Xiano, S.J., Textor, M., Spencer, N.D. , Wieland, M., Keller, B. and Sigrist, H.: Immobilization of the cell-adhesive peptide Arg-Gly-Asp-Cys (RGDC) on titanium surfaces by covalent chemical attachment. J. Mater Sci : Mater Med. 8, 867-872, 1997.), a method in which a polymeric material with biological absorptivity is bonded on the surface of titanium or the titanium alloy after carrying out the silane treatment (for example, refer to Bearinger, J.P., Castner, D.G., Golledge, S.L., Rezania, A. , Hubchak, S. and Healy, K.E. : P (AAm-co-EG) interpenetrating networks grafted to oxide surfaces: Surface characterization, protein adsorption, and cell detachment studies. Langmuir 13, 5175-5183, 1997.), a method in which the peptide is bonded on the surface of titanium or the titanium alloy after carrying out a gold evaporation deposition (for example, refer to Ferris, D.M., Moodie, G.D., Dimond, P.M., Gioranni, C.W., Ehrlich, M.G, and Valentini, R.F.: RGD-coated titanium implants stimulate increased bone formation in vivo. Biomaterials 20, 2323-2331, 1999.), and a method in which the peptide is bonded on the surface of titanium or the titanium alloy after coating the polymeric material with biological absorptivity (for example, refer to Kenausis, G.L., Voeroes, J., Elbert, D.L., Huang, N., Hofer, R., Ruiz-taylor, L., Textor, M., Hubbell, J.A. and Spencer, N.D.: Poly(L-lysine)-g-poly (ethylene glycol) layers on metal surfaces: attachment mechanism and efforts of polymer architecture on resistance to protein adsorption. J. Phy Chem. B 104, 3298-3309, 2000.). However, these methods have problems that these treating processes are complicated and unstable since the only surface is coated and have poor chemical bonding.

Further, CN-A-1 451 367 discloses an artificial tooth root material with induced osteoplastic bioactivity. Puelo et al. ("A technique to immobilize bioactive proteins, including bone morphogenetic protein-4 (BMP-4), Biomaterials 23 (2002) 2079-2087) disclose the use of plasma-treated metallic biomaterials for retaining biologically active proteins. US patent application 2003/0135282 A1 discloses a method used in surface treatment of implants and protheses and GB-A-1 401 233 discloses a method for modifying the surfaces of articles formed from polymers containing free hydroxyl.

The primary objective of the present invention is to provide the titanium or titanium alloy implant obtainable by a process, in which the surface modification is carried out, and the surface treating method thereof, whereby the bone bonding can be achieved easily and certainly as compared with the conventional surface modification of the titanium or titanium alloy implant, in which the treatment is complicated and unstable.

The earnest work was carried out in order to solve the above-mentioned problems and, as a result of this, the followings were found out to complete the invention. When the surface of titanium or the titanium alloy is directly modified by way of apatite being deposited or adsorbed on the surface of titanium or the titanium alloy, or the biofunctional protein or the peptide being used as the cell bonding factor, the cell bonding factor is easily peeled from the surface of titanium or the titanium alloy in a living body. Further, even when the cell bonding factor is bonded previously with an objective cell, the cell bonding factor and the cell are peeled together from the material surface, so that the sufficient effect of the surface modification of titanium or the titanium alloy may not be exerted in the living body. Thus, it was considered that the bone bonding could be made in the early stage when basic fibroblast growth factor (bFGF) was applied to the surface of the implant made with titanium or the titanium alloy. The basic fibroblast growth factor (bFGF) has an important role for the bone bonding. In order to efficiently bond the basic fibroblast growth factor (bFGF) with the surface of the titanium or titanium alloy implant, the surface treatment with a polyphosphoric acid is the most effective. Then, the invention was completed.

The present invention relates to a titanium or titanium alloy implant obtainable by a process in which the surface is treated with the polyphosphoric acid and the basic fibroblast growth factor (bFGF), the surface treating method of the titanium or titanium alloy implant comprising treating of the surface of the titanium or titanium alloy implant with a polyphosphoric acid solution having a concentration of 0.05 to 70 % by weight, and treating thereafter of the surface of the implant with a basic fibroblast growth factor (bFGF) solution having a concentration of 1 to 500 ng/ml, and the other surface treating method of the titanium or titanium alloy implant comprising treating of the surface of the titanium or titanium alloy implant with a mixed solution in which the concentration of the polyphosphoric acid solution is 0.05 to 70 % by weight and the concentration of the basic fibroblast growth factor (bFGF) is 1 to 500 ng/ml.

Further, in the surface treating method of the titanium or titanium alloy implant according to the present invention, it is preferable that the concentration of the polyphosphoric acid is 0.05 to 5 % by weight, and the concentration of the basic fibroblast growth factor (bFGF) is 10 to 100 ng/ml.

According to the present invention of the implant titanium or titanium alloy implant and the surface treating method thereof, the surface modification is carried out so as to make the bone bonding easily and certainly as compared with the conventional complicated and unstable surface modification of the titanium or titanium alloy implant.

The titanium or titanium alloy implant in the present invention means a formed body, which is formed with titanium or the titanium alloy and used in the living body. As for the titanium or titanium alloy implant, if the implant has a physical characteristic and safety which are needed for using in the living body, a form or usage type is not limited especially. For example, as an artificial bone metal material, a material having the forms such as a column form, a plate form, a block form, a sheet form, a fiber form, a pellet form or the like can be used arbitrary. Further, the implant may be of product types such as a stem material for an artificial hip joint, a bone replacement material, an artificial corpus vertebrae, an artificial tooth root, artificial intervertebral disc, a bone plate, a bone screw, or the like.

The basic fibroblast growth factor (bFGF) used in the present invention is a commonly known polypeptide, having a specific sequence with the number of amino acid residues being 146, which is indicated in Japanese Patent PublicationNo. 07-89936 "Recombined Fibroblast Growth Factor", Japanese Patent Publication No. 08-29097 "Fibroblast Growth Factor", Japanese Patent No. 2619208 "Recombined Fibroblast Growth Factor", Japanese Patent No. 2761513 "Recombined Fibroblast Growth Factor", or the like. The polypeptide is a cell division- promoting substance, which is effective to a normal diploid fibroblast or a cell line. As the basic fibroblast growth factor (bFGF), the generally used basic fibroblast growth factor (bFGF) can be used other than a natural basic fibroblast growth factor (bFGF) existing in a pituitary. For example, the basic fibroblast growth factor (bFGF) made by synthesizing by a method indicated in Japanese Patent Publication No. 08-29097 can be used.

As for the surface treating method of the titanium or titanium alloy implant according to the present invention, it is preferable that the surface of the titanium or titanium alloy implant is washed before treatment with the polyphosphoric acid and the basic fibroblast growth factor (bFGF). As for the washing, an acid treatment is preferable, and it is preferable that the washing is carried out with at least one kind of a solution selected from an aqueous solution of sodium peroxodisulfate (Na₂S₂O₈) having the concentration of 0.05 to 5 % by weight, a sulfuric acid (H₂SO₄) aqueous solution being 0.01N or more, and a hydrochloric acid (HCl) aqueous solution being 0.01N or more. Especially, a case of treating with the hydrochloric acid is the most preferable, since the bonding to titanium surface of a phosphorylated amino acid or a phosphorylated peptide is remarkably increased.

After washing of the surface by the acid treatment as mentioned above if necessary, the surface treatment is carried out with the polyphosphoric acid and the basic fibroblast growth factor (bFGF). As for the polyphosphoric acid, the solution having the concentration of 0.05 to 70 % by weight is preferably applied to the implant surface. If the concentration of the solution is less than 0.05 % by weight, the effect of the surface treatment with the polyphosphoric acid is hardly obtained. On the other hand, if the concentration is more than 70 % by weight, the effect of the surface treatment is decreased. The most preferable concentration range of the polyphosphoric acid in the treatment solution is 0.05 to 5 % by weight.

As for the basic fibroblast growth factor (bFGF), the solution having the concentration of 1 to 500 ng/ml is preferably applied to the surface of the titanium or titanium alloy implant. If the concentration of the basic fibroblast growth factor (bFGF) is less than 1 ng/ml, the effect of the surface treatment is hardly obtained. Even if the concentration is more than 500 ng/ml, the effect is not increased more, so that the efficiency of the surface treatment is decreased. The most preferable concentration range of the basic fibroblast growth factor (bFGF) in the treatment solution is 10 to 100 ng/ml.

More particularly, the surface treating method of the titanium or titanium alloy implant according to the present invention comprises, treating of the surface of the titanium or titanium alloy implant with the polyphosphoric acid solution having the concentration of 0.05 to 70 % by weight, and treating thereafter with the basic fibroblast growth factor (bFGF) solution having the concentration of 1 to 500 ng/ml. Further, in the surface treating method of the titanium or titanium alloy implant according to the present invention, the surface treatment may be carried out with the polyphosphoric acid and the basic fibroblast growth factor (bFGF) simultaneously, using the mixed solution, in which the concentration of the polyphosphoric acid is 0.05 to 70 % by weight and the concentration of the basic fibroblast growth factor (bFGF) is 1 to 500 ng/ml.

In addition, the treatment with the polyphosphoric acid, the treatment with the basic fibroblast growth factor (bFGF), or the treatment with the mixed solution of the polyphosphoric acid and the basic fibroblast growth factor (bFGF) may be carried out dividedly in several steps. However, when the surface treatment is carried out with two kinds of the solutions of the polyphosphoric acid and the basic fibroblast growth factor (bFGF), where the concentration of the polyphosphoric acid is 0.05 to 70 % by weight and the concentration of the basic fibroblast growth factor (bFGF) is 1 to 500 ng/ml, it is necessary to contact the polyphosphoric acid on the surface of the implant at first in order to form a coating layer mainly comprising the polyphosphoric acid, since the polyphosphoric acid has the excellent adhesibility with titanium or the titanium alloy.

As for the polyphosphoric acid solution having the concentration of 0.05 to 70 % by weight, the basic fibroblast growth factor (bFGF) solution having the concentration of 1 to 500 ng/ml, and the mixed solution in which the concentration of the polyphosphoric acid is 0.05 to 70 % by weight and the concentration of the basic fibroblast growth factor (bFGF) is 1 to 500 ng/ml, it is preferable that these solutions are aqueous solutions. As the surface treating method of the titanium or titanium alloy implant, although coating, spraying or the like canbe indicated, a dipping method, in which the titanium or titanium alloy implant is dipped in the solution, can obtain a good effect with the highest efficiency.

When the surface treatment is carried out, although it can be carried out within the range of 0 to 120°C, it is preferably carried out at 42°C or less because thermal denaturation of the basic fibroblast growth factor (bFGF) is considered. The treatment at less than 10°C is not realistic from the point of the efficiency of the surface treatment. More preferably, the surface treatment is carried out at 25 to 37°C. More particularly, when the surface treatment is carried out by dipping in the polyphosphoric acid solution and the basic fibroblast growth factor (bFGF) solution, it is preferable that the dipping treatment with the polyphosphoric acid is carried out at 25 to 37°C for 0.5 to 48 hours, and the dipping treatment with the basic fibroblast growth factor (bFGF) is carried out at 25 to 37°C for 0.01 to 48 hours.

### [Example]

Hereinafter, the present invention is explained concretely with examples, but the present invention is not limited to these examples.

### [Examples 1 to 6 and Comparison example 1]

A dental titanium alloy disc made by GC Corporation and having a diameter of 5.8 mm and a thickness of 5 mm was used as an implant material of titanium or the titanium alloy. The dental titanium alloy disc was subjected to an ultrasonic washing treatment for 30 minutes in ultrapure water, where, in cases of Examples 5 and 6, the titanium alloy disc was subjected to the ultrasonic washing treatment in a solution of 1N hydrochloric acid and thereafter an ultrasonic washing for 30 minutes in ultrapure water. The hydrochloric acid was made by Katayama Chemical Corporation. The titanium alloy disc was subjected to the surface treatment by dipping at 37°C for 24 hours in aqueous solutions of 0.1 % by weight, 1 % by weight and 10 % by weight of polyphosphoric acid. The polyphosphoric acid was made by Wako Pure Chemical Industries, Ltd. After that, the titaniumalloydiscwas subjected to the surface treatment by dipping for 0.2 hours in aqueous solutions of 10ng/ml and 50ng/ml of basic fibroblast growth factor (bFGF). Further, as Comparison example 1, the titanium alloy disc was subjected to the ultrasonic washing treatment for 30 minutes in the solution of 1N hydrochloric acid (made by Katayama Chemical Corp.) and thereafter the ultrasonic washing for 30 minutes in the ultrapure water, without the treatment with the polyphosphoric acid and the basic fibroblast growth factor (bFGF), and as Comparison example 2, the titanium alloy disc, which was the same disc as that of Example 1, was subjected to the surface treatment by only dipping at 37°C for 24 hours in the 1 % by weight polyphosphoric acid solution.

### Examples 7 to 12

A titanium alloy disc like that of Example 1 was washed and treated like Examples 1 to 4, where in cases of Examples 1 and 12, the disc was subjected to the ultrasonic washing treatment for 30 minutes in the aqueous solution of 1N hydrochloric acid (made by Katayama Chemical Corp.) and thereafter the ultrasonic washing treatment for 30 minutes in the ultrapure water. The titanium alloy disc was subjected to the surface treatment by dipping at 37°C for 24 hours in a mixed solutions of 0.1 % by weight, 1 % by weight and 10 % by weight polyphosphoric acids (made by Wako Pure Chemical Ind. Ltd.) and 10ng/ml and 50ng/ml basic fibroblast growth factors (bFGF).

### <Adhesion test of a mesenchymal stem cell originated from a human bone marrow >

Cell adhesion of a mesenchymal stem cell originated from a human bone marrow (hMSC) to the surface of the titanium alloy disc subjected to the surface treatment with the polyphosphoric acid and the basic fibroblast growth factor (bFGF), was evaluated. After fixing the titanium alloy discs with 24 well plates using O-ring, 10⁵/well of the mesenchymal stem cell originated from the human bone marrow (hMSC) were seeded on the titanium alloy discs and cultivated using a serum-free medium (Dulbecco' s Modified Eagle's Medium made by Sigma Corporation). The number of cells adhered to the surface of the titanium alloy disc was evaluated after 3 hours using MTS Assay. These results were shown in Table 1. As for the titanium alloy discs subjected to the surface treatment with the polyphosphoric acid and the basic fibroblast growth factor (bFGF), the cell adhesion was clearly increased as compared with the untreated titanium alloy disc. Therefore, predominancy of the present invention could be confirmed.

| | Example 1 | Example 2 | Example 3 | Example 4 | Example 5 | Example 6 | Example 7 | Example 8 | Example 9 | Example 10 | Example 11 | Example 12 | Comparison 1 | Comparison 2 |
|---|---|---|---|---|---|---|---|---|---|---|---|---|---|---|
| Polyphosphoric Acid % by weight | 0.1 | 0.1 | 1 | 1 | 10 | 10 | 0.1 | 0.1 | 1 | 1 | 10 | 10 | - | 1 |
| bFGF Concentration ng/ml | 10 | 50 | 10 | 50 | 10 | 50 | 10 | 50 | 10 | 50 | 10 | 50 | - | - |
| Relative Evaluation by MTS Assay | 1.19 | 1.25 | 1.22 | 1.30 | 1.20 | 1.24 | 1.21 | 1.30 | 1.26 | 1.31 | 1.25 | 1.23 | 0.91 | 1.00 |

## Claims

1. Titanium or titanium alloy implant obtainable by a process wherein a surface is treated with polyphosphoric acid and basic fibroblast growth factor (bFGF).

2. A surface treating method of a titanium or titanium alloy implant, the method comprising, treating with a polyphosphoric acid solution having a concentration of 0.05 to 70 % by weight, and treating with a basic fibroblast growth factor (bFGF) solution having a concentration of 1 to 500 ng/ml.

3. A surface treating method of a titanium or titanium alloy implant, the method comprising, treating with a mixed solution of polyphosphoric acid having a concentration of 0.05 to 70 % by weight and basic fibroblast growth factor (bFGF) having a concentration of 1 to 500 ng/ml.

4. The surface treating method of a titanium or titanium alloy implant according to claim 2 or 3,
wherein the concentration of polyphosphoric acid is 0.05 to 5 % by weight.

5. The surface treating method of a titanium or titanium alloy implant according to any one of claim 2 to claim 4,
wherein the concentration of basic fibroblast growth factor (bFGF) is 10 to 100 ng/ml.

## Patentansprüche

1. Implantat aus Titan oder einer Titanlegierung, durch ein Verfahren erhältlich, bei dem eine Oberfläche mit Polyphosphorsäure und basischem Fibroblastenwachstumsfaktor (bFGF) behandelt wird.

2. Oberflächenbehandlungsverfahren eines Implantats aus Titan oder einer Titanlegierung, das Verfahren umfassend das Behandeln mit einer Polyphosphorsäurelösung, die eine Konzentration von 0,05 bis 70 Gewichts-% aufweist, und das Behandeln mit einer Lösung von basischem Fibroblastenwachstumsfaktor (bFGF), die eine Konzentration von 1 bis 500 ng/ml aufweist.

3. Oberflächenbehandlungsverfahren eines Implantats aus Titan oder einer Titanlegierung, das Verfahren umfassend das Behandeln mit einer gemischten Lösung von Polyphosphorsäure, die eine Konzentration von 0,05 bis 70 Gewichts-% aufweist, und basischem Fibroblastenwachstumsfaktor (bFGF), der eine Konzentration von 1 bis 500 ng/ml aufweist.

4. Oberflächenbehandlungsverfahren eines Implantats aus Titan oder einer Titanlegierung nach Anspruch 2 oder 3, wobei die Konzentration der Polyphosphorsäure 0,05 bis 5 Gewichts-% ist.

5. Oberflächenbehandlungsverfahren eines Implantats aus Titan oder einer Titanlegierung nach einem der Ansprüche 2 bis 4, wobei die Konzentration des basischen Fibroblastenwachstumsfaktors (bFGF) 10 bis 100 ng/ml ist.

## Revendications

1. Implant en titane ou en alliage de titane pouvant être obtenu par un procédé dans lequel une surface est traitée avec du poly(acide phosphorique) et avec un facteur de croissance de fibroblastes basique (bFGF).

2. Procédé de traitement de surface d'un implant en titane ou en alliage de titane, le procédé comprenant le traitement avec une solution de poly(acide phosphorique) ayant une concentration de 0,05 à 70 % en poids, et le traitement avec une solution de facteur de croissance de fibroblastes basique (bFGF) ayant une concentration de 1 à 500 ng/ml.

3. Procédé de traitement de surface d'un implant en titane ou en alliage de titane, le procédé comprenant le traitement avec une solution mixte de poly (acide phosphorique) ayant une concentration de 0,05 à 70 % en poids et de facteur de croissance de fibroblastes basique (bFGF) ayant une concentration de 1 à 500 ng/ml.

4. Procédé de traitement de surface d'un implant en titane ou en alliage de titane selon la revendication 2 ou 3,
dans lequel la concentration du poly(acide phosphorique) est de 0,05 à 5 % en poids.

5. Procédé de traitement de surface d'un implant en titane ou en alliage de titane selon l'une quelconque des revendications 2 à 4,
dans lequel la concentration du facteur de croissance de fibroblastes basique (bFGF) est de 10 à 100 ng/ml.
